# EUROPEAN PATENT APPLICATION

(11) **EP 1 770 437 A1**
(43) Date of publication of application: **04.04.2007**
(21) Application number: 05108321.0
(22) Date of filing: 12.09.2005
(51) Int. Cl.: G03B 42/04, G06F 19/00

(54) **Image processing method**

(71) Applicant: AGFA-GEVAERT, 2640 Mortsel (BE)
(72) Inventor: Milicevic, Sasha AGFA-GEVAERT, 2640 Mortsel (BE)

(57) **Abstract**

A method for processing a medical image represented by a digital image signal and accompanying image identification data wherein upon processing of one of the image and the image identifying data, a corresponding change of the other is effectuated.

## Description

### FIELD OF THE INVENTION

The present invention relates to an image processing method and system for use in the field of processing of digital medical images.

### BACKGROUND OF THE INVENTION

Within the context of digital medical imaging techniques, variants can be distinguished: CR (Computed Radiography), DR (Digital Radiography), CT (Computed Tomography), MRI (Magnetic Resonance Imaging) etc. These medical imaging techniques generate a radiation image of a subject or a region of the subject through the use of a so-called 'image acquisition device', e.g. a CT scanner, an X-ray modality. The image data acquired by these acquisition devices can then be distributed to other devices, e.g. workstations, archiving stations, display stations etc. The present invention relates to the medical image data acquired with acquisition devices as described higher and to the processing that is applied to these image data.

For example in CR (computed radiography) an X-ray image of a body part of a patient is recorded on a recording medium, more particularly on a photostimulable phosphor screen. The photostimulable phosphor screen is conveyed in a cassette.

The cassette carrying the exposed recording medium, e.g. the exposed photostimulable phosphor screen, is then fed into a digitizing apparatus where the recorded image is read out by scanning the screen with stimulating radiation of the appropriate wavelength emitted by a laser and by detecting the image-wise modulated light emitted by the screen upon said stimulation.
This image-wise modulated light is detected by a photoelectric converter and converted into an electric signal representation of the radiation image.

This electric signal representation is then digitized and can then be applied to a workstation on which image processing software is installed and/or to an archiving station that allows storing of the medical image and/or a hard copy recorder that allows medical images to be printed as a hard copy.

Generally the above described devices are interconnected by a network so that image data can be sent directly from one device to another. Alternatively the devices are not interconnected and the data can be imported into one device after being exported from another one.

An image, part of an examination, can be defined by parameters that describe the condition under which the image was taken. Relevant parameters in this context are for example in case of extremities an indication of a left or right extremity (e.g. left fore arm). Another parameter is the view position. This parameter indicates whether the image was acquired from the lateral side of the patient (left or right) or the frontal side (dorsal or ventral).

In the case of a computed radiography image that is recorded on a photostimulable phosphor screen conveyed in a cassette, these parameters are identified and written into a tag for example a radio-frequency tag, provided on the cassette conveying the screen.

In the digitizer these data are read out from the identification tag and associated with the read out image signal representing the image to which they pertain. These data are transferred to peripheral devices (processing unit, display station, archive station, etc.) together with the image signal.

It may happen that a cassette has been identified with data regarding an exposure view position which does not correspond with the effectively applied exposure view position. For example in a situation in which a chest PA recording has been planned while at patient arrival a chest AP is recorded on the recording medium, it may happen that the identification tag on the cassette comprises data indicative of a PA recording whereas the recording medium carries a chest PA image.

Image data and associated, erroneous identification data are then transferred to a processing and display system.

In current image processing and display systems, a AP image identified as PA image will be flipped around the vertical axis by a pre-processing algorithm.
Since this does not represent the true situation at image recording, this action might result in an incorrect image interpretation and consequentially also in an incorrect diagnosis.
While in such a case the image may be flipped back to its original status by operator intervention, the data indicative of the view position remain unaffected.

It is an object of the present invention to provide a image processing method and a corresponding image processing device that does not have the drawbacks of the state of the art.

### SUMMARY OF THE INVENTION

The above-mentioned advantageous effects are realized by a method and a device as set out in the appending claims.

The invention provides a method for processing a medical image represented by a digital image signal and by accompanying image identification data such as data describing specifications of the exposure or the examination conditions, etc. Upon modification of one of the display of said image and the image identification data, a corresponding change of the other is caused.

The invention further provides a processing and display device comprising means for displaying an image represented by a digital image signal,
means for displaying image identification data and
means for processing said image. The processing means are arranged to effectuate upon modification of one of the display of said image and the image identifying data, a corresponding change of the other.

Specific features for preferred embodiments of the invention are set out in the dependent claims.

Further advantages and embodiments of the present invention will become apparent from the following description.

### DETAILED DESCRIPTION OF THE INVENTION

In an image acquisition phase a patient is positioned in between an X-ray source and a recording medium. In the described example the recording medium is a photostimulable phosphor screen conveyed in a cassette. Alternative recording media may be envisaged such as photosensitive film, direct x-ray detectors etc.

X-rays emitted by the x-ray source will encounter the patient who is positioned between the source and the cassette conveying the recording medium and, depending on the material the x rays encounter (tissue, bone, skin etc.), the x-rays will penetrate the material differently. The differential penetration of the x-rays will be reflected in the image that is stored in the photostimulable phosphor screen in the cassette.

The cassette is provided with an identification means, e.g. a radiofrequency tag in which demographic data such as patient identifying data (name, gender, date of birth, pathology history etc.) and data identifying the examination and the examination conditions are written.

The data regarding the examination conditions describe the details of the image acquisition, e.g. patient facing/not facing X-ray source, landscape or portrait position of cassette during exposure, body part that is examined, etc.

In the described specific embodiment these data are written into the tag in an identification device through radiofrequency transmission. Alternative identification means requiring other types of communication may be envisaged. An example of another type of device is an EEPROM requiring data communication through galvanic contacts.

Once the image is generated on the photostimulable phosphor screen, the cassette conveying an exposed screen and an identification tag are fed into a so-called digitizer.
There the image data and the data stored in the identification tag are read out.

For this purpose the photostimulable phosphor screen carrying the radiation image is scanned with stimulating laser light of the appropriate wavelength. Image-wise modulated light emitted by the screen upon stimulation is detected by a photoelectric converter and converted into an electric signal representation of the radiation image. This electric signal representation is digitized.

The data stored in the identification tag are also read out.

Once the image is read out of the recording medium and the identification data are read out of the identification tag, the image data and associated identification data will be send over a communication line, in the described case over a DICOM connection towards a peripheral device such as a processing and display unit and/or an archive station or the like.

In order to enable this transmission, digitizer and peripheral station have to be configured so as to be aware of each others presence.

The DICOM protocol is a protocol used on top of a TCP/IP protocol. Both instances communicate over a DICOM protocol through the TCP/IP protocol. This implies that both instances are configured according to the requirements of these protocols.

For TCP/IP, both instances should have a host name, IP address and subnet mask configured. In order to use the DICOM protocol, both instances should have a so called AE Title configured. When all parameters have been defined for both protocols, communication between the instances is possible.

The data will be transmitted as binary data during a message exchange sequence between both instances. When the data has been transmitted by the digitizer and these data has been received successfully by the image processing station, an affirmation will be sent to the digitizer to inform the latter that the data transmission has succeeded.

When the data have been sent to the image processing and display station, the latter will store these data on its local hard disc in a proprietary format. Once the data is present on the local hard disc of the image processing and display station, it is available to the medical image processing software so that modifications can be performed on the image.

Through the graphical user interface of the medical image processing software, the operator can browse through all the available images, i.e. the images that are available on the hard disc. Consequently, he can choose to select a specific image and open it for display. This action will cause the software package to access the data (e.g. examination and/or demographic data) related to the selected image and display the data on a screen.

When an image is selected, all the data related to the image will be retrieved from the hard disc and displayed on screen. Consequently, the operator can modify the data as described above.

Without the provisions of the present invention the image could be manipulated by the operator so that the displayed conditions do not longer match the actual presentation state of the image. Vice versa, the described conditions could also be manipulated through the software package introducing a contradiction relative to the image presentation state. The present invention provides a so-called "synchronization" of both types of information, i.e. the actual image presentation state and the described examination conditions.

The invention provides that for example when the image is flipped over the vertical axis by the operator, the application will automatically adjust the corresponding parameter within the described examination conditions. By performing this so-called synchronization, there will always be a perfect match between the image presentation state and the examination conditions.

For certain actions, e.g. flipping an image, the software provides that the relevant parameters in the examination description require up-dating. For other actions, e.g. altering the described examination conditions, the software application provides that the image presentation state of the image is to be altered. A set of applicable rules are thus coded into the software application.

The altered situation is displayed on the screen and/or replaces the former situation in the memory of the image processing and display device.

According to a specific embodiment of the present invention the operator will not indicate the laterality parameter during the image acquisition phase, leaving this parameter empty. During the image processing phase, the operator can add a digital marker to the image to indicate laterality. This digital marker, e.g. a digital left or right indicator, is implemented as a predefined electronic annotation that is placed on the image when it is displayed on the screen. The electronic annotation will be associated with the image and stored.

It will give a visual indication of the lateral side that is at stake. Furthermore, this digital marker serves as a trigger for the software application to deduct all parameters that are related to the laterality. Consequently the application has all relevant parameters available and can start processing the image accordingly.

In still another embodiment of the present invention an automatic flip of an image is provided in case this action is configured on the system level. In contrast to the first aspect of the invention under scope, this automatic flipping will not be reflected in the described examination conditions. This is because this automatic flip is only intended for presentation purposes and thus needs not to be coded in the described examination conditions.

When the application is confronted with an image for which this automatic flipping was requested (during the configuration phase of the application), it will automatically perform this flipping around the vertical axis. The image is flipped, without any user interaction.

Having described in detail preferred embodiments of the current invention, it will now be apparent to those skilled in the art that numerous modifications can be made therein without departing from the scope of the invention as defined in the appending claims.

## Claims

1. A method for processing and displaying a medical image represented by a digital image signal and accompanying image identification data **characterised in that** upon processing one of the image and the image identifying data, a corresponding change of the other is caused.

2. A method according to claim 1 wherein the result of said corresponding change is displayed.

3. A method according to claim 1 wherein the result of said corresponding change is stored in memory.

4. A signal processing and display device comprising means for displaying an image represented by a digital image signal,
means for displaying image identification data,
means for processing said image,
**characterised in that** said means for processing are arranged to effectuate upon processing one of said image and the image identifying data, a corresponding change of the other.

5. A device according to claim 4 arranged to display the result of said corresponding change.

6. A device according to claim 4 comprising a memory wherein said corresponding change is stored.
